# EUROPÄISCHE PATENTSCHRIFT

(11) **EP 1 761 962 B1**
(45) Veröffentlichungstag und Bekanntmachung des Hinweises auf die Patenterteilung: **03.02.2010**
(21) Anmeldenummer: 05753649.2
(22) Anmeldetag: 22.06.2005
(51) Int. Cl.: H01L 51/30, C07C 211/43

(54) **ORGANISCHE ELEKTROLUMINESZENZVORRICHTUNG**
ORGANIC ELECTROLUMINESCENT DEVICE
DISPOSITIF ELECTROLUMINESCENT ORGANIQUE

(30) Priorität: 26.06.2004 DE 102004031000
(43) Veröffentlichungstag der Anmeldung: 14.03.2007
(73) Patentinhaber: Merck Patent GmbH, 64293 Darmstadt (DE)
(72) Erfinder: VESTWEBER, Horst, 34630 Gilserberg (DE); STÖSSEL, Philipp, 60487 Frankfurt (DE); GERHARD, Anja, 64291 Darmstadt (DE); PARHAM, Amir, 65929 Frankfurt (DE)
(86) Internationale Anmeldenummer: PCT/EP2005/006727
(87) Internationale Veröffentlichungsnummer: WO 2006/000388

(56) Entgegenhaltungen:
- EP-A- 1 074 601
- US-A- 4 769 302
- US-A1- 2002 058 156
- PATENT ABSTRACTS OF JAPAN Bd. 2003, Nr. 12, 5. Dezember 2003 (2003-12-05) & JP 2003 248331 A (KONICA CORP), 5. September 2003 (2003-09-05)
- PATENT ABSTRACTS OF JAPAN Bd. 1998, Nr. 14, 31. Dezember 1998 (1998-12-31) & JP 10 255979 A (MINOLTA CO LTD), 25. September 1998 (1998-09-25)
- PATENT ABSTRACTS OF JAPAN Bd. 1996, Nr. 05, 31. Mai 1996 (1996-05-31) & JP 08 012969 A (TDK CORP), 16. Januar 1996 (1996-01-16)
- PATENT ABSTRACTS OF JAPAN Bd. 1995, Nr. 10, 30. November 1995 (1995-11-30) & JP 07 175236 A (KONICA CORP), 14. Juli 1995 (1995-07-14)
- PATENT ABSTRACTS OF JAPAN Bd. 017, Nr. 170 (P-1515), 31. März 1993 (1993-03-31) & JP 04 330452 A (KONICA CORP), 18. November 1992 (1992-11-18)
- PATENT ABSTRACTS OF JAPAN Bd. 016, Nr. 499 (E-1280), 15. Oktober 1992 (1992-10-15) & JP 04 184892 A (RICOH CO LTD), 1. Juli 1992 (1992-07-01)
- PATENT ABSTRACTS OF JAPAN Bd. 013, Nr. 347 (P-910), 4. August 1989 (1989-08-04) & JP 01 106070 A (KONICA CORP), 24. April 1989 (1989-04-24)
- PATENT ABSTRACTS OF JAPAN Bd. 013, Nr. 185 (P-865), 2. Mai 1989 (1989-05-02) & JP 01 013553 A (KONICA CORP), 18. Januar 1989 (1989-01-18)
- PATENT ABSTRACTS OF JAPAN Bd. 012, Nr. 431 (P-786), 15. November 1988 (1988-11-15) & JP 63 163361 A (CANON INC), 6. Juli 1988 (1988-07-06)
- PATENT ABSTRACTS OF JAPAN Bd. 013, Nr. 347 (P-910), 4. August 1989 (1989-08-04) & JP 01 105955 A (KONICA CORP), 24. April 1989 (1989-04-24)

## Beschreibung

Die vorliegende Erfindung beschreibt den Einsatz bestimmter Verbindungen in organischen Elektrolumineszenzvorrichtungen.

Der Einsatz halbleitender organischer Verbindungen, die zur Emission von Licht im sichtbaren Spektralbereich befähigt sind, in organischen Elektrolumineszenzvorrichtungen (OLEDs) steht gerade am Anfang der Markteinführung. Der allgemeine Aufbau organischer Elektrolumineszenzvorrichtungen ist beispielsweise in US 4539507, US 5151629, EP 0676461 und WO 98/27136 beschrieben. Für einfache OLEDs enthaltende Vorrichtungen ist die Markteinführung bereits erfolgt, wie die Autoradios der Firma Pioneer, die Mobiltelefone der Firmen Pioneer und SNMD oder eine Digitalkamera der Firma Kodak mit "organischem Display" belegen. Weitere derartige Produkte stehen kurz vor der Einführung.

Allerdings zeigen diese Vorrichtungen immer noch erhebliche Probleme, die einer dringenden Verbesserung bedürfen:
1. Die operative Lebensdauer ist insbesondere bei blauer Emission immer noch gering, so dass bis dato nur einfache Anwendungen kommerziell realisiert werden konnten.
2. Die Effizienz ist in den letzten Jahren verbessert worden, ist aber gerade bei fluoreszierenden OLEDs immer noch zu niedrig und muss verbessert werden.
3. Die Betriebsspannung ist gerade bei fluoreszierenden OLEDs recht hoch und sollte daher weiter verringert werden, um die Leistungseffizienz zu verbessern. Das ist insbesondere für mobile Anwendungen von großer Bedeutung.
4. Viele blau emittierende Emitter, die sowohl aromatische Amine, wie auch Doppelbindungssysteme enthalten, sind thermisch nicht stabil und zersetzen sich beim Sublimieren oder beim Aufdampfen. Dadurch ist die Verwendung dieser Systeme nicht bzw. nur unter großen Verlusten und mit hohem technischen Aufwand möglich.

Als Stand der Technik kann die Verwendung bestimmter Arylvinylamine, die an der Doppelbindung des stilbenartigen Systems nicht substituiert sind, von Idemitsu genannt werden (z. B. WO 04/013073, WO 04/016575, WO 04/018587). Damit werden sehr gute Lebensdauern bei tiefblauer Emission zitiert. Allerdings sind diese Ergebnisse stark abhängig vom verwendeten Hostmaterial, so dass die zitierten Lebensdauern nicht als Absolutwerte verglichen werden können, sondern immer nur bei Einsatz in einem optimierten System. Weiterhin sind diese Verbindungen thermisch instabil und lassen sich nicht unzersetzt verdampfen, was einen hohen technischen Aufwand für die Aufdampfung erfordert und somit einen deutlichen technischen Nachteil darstellt. Ohne an eine bestimmte Theorie gebunden sein zu wollen, vermuten wir, dass die thermische Instabilität zumindest teilweise darauf beruht, dass die Verbindungen thermische cis-trans-Isomerisierung zeigen können. Ein weiterer Nachteil ist die Emissionsfarbe dieser Verbindungen. Während Idemitsu tiefblaue Emission (CIE-y-Koordinaten im Bereich von 0.15-0.18) zitiert, konnten diese Farbkoordinaten nicht in einfachen Devices gemäß dem Stand der Technik reproduziert werden. Im Gegenteil erhält man hier grünblaue Emission. Es ist nicht offensichtlich, wie mit diesen Verbindungen blaue Emission erzeugt werden kann.

Die Verwendung aryl-substituierter Tristilbenamine in OLEDs ist in der Literatur bereits beschrieben:
In EP 1074601 wird die Verwendung von Aminostilbenverbindungen in organischen Elektrolumineszenzvorrichtungen beschrieben. Gegenstand der Erfindung sind jedoch ausschließlich Vorrichtungen enthaltend diarylaminosubstituierte Verbindungen.
Weiterhin sind in US 2002/058156 Elektrolumineszenzvorrichtungen enthaltend cyclohexylidensubstituierte Aminostilbenverbindungen offenbart.
In JP 10-255979 wird die Verwendung verschiedener unsubstituierter und substituierter Arylvinylamine in der Lochinjektionsschicht von OLEDs beschrieben. Die Verwendung dieser Verbindungen als emittierende Materialien geht aus der Beschreibung nicht hervor.
In JP 2913116 wird die Verwendung unsubstituierter und substituierter Tristilbenamine und anderer Arylvinylverbindungen in OLEDs beschrieben. Dabei werden diese Verbindungen als Reinsubstanz in der emittierenden Schicht eingesetzt. Es wird zwar beschrieben, dass diese Vorrichtungen "schön leuchten", aber das Fehlen quantitativer Device-Daten deutet darauf hin, dass Effizienz, Betriebsspannung, Lebensdauer und Farbe nicht zufriedenstellend sind. Weiterhin wird beschrieben, dass jedes Kohlenstoffatom der Doppelbindung bevorzugt nur einfach substituiert ist und dass also beispielsweise einfache Stilben-Derivate vor Triarylethen-Derivaten bevorzugt sind. Aus der Beschreibung geht nicht hervor, dass sich arylsubstituierte Tristilbenamin-Derivate besonders und besser als unsubstituierte Derivate für die Verwendung in OLEDs eignen könnten. Insbesondere lässt sich nicht erkennen, in welcher Form bzw. in welchem Device-Aufbau außer dem beschriebenen (Verwendung als Reinsubstanz in der emittierenden Schicht) solche Verbindungen besonders nutzbringend eingesetzt werden könnten.

Es besteht also weiterhin Bedarf an blau emittierenden Verbindungen, die in organischen Elektrolumineszenzvorrichtungen zu guten Effizienzen und gleichzeitig zu hohen Lebensdauern führen und die technisch unproblematisch zu verarbeiten sind. Es wurde nun überraschend gefunden, dass organische Elektrolumineszenzvorrichtungen, die bestimmte - im Folgenden aufgeführte - Verbindungen als blau emittierende Dotanden in einem Hostmaterial enthalten, deutliche Verbesserungen gegenüber dem Stand der Technik aufweisen. Mit diesen Materialien ist es möglich, gleichzeitig hohe Effizienzen und lange Lebensdauern zu erhalten. Außerdem lassen sich diese Verbindungen im Gegensatz zu Materialien gemäß dem Stand der Technik ohne merkliche Zersetzung sublimieren und aufdampfen und sind daher deutlich leichter zu handhaben als Materialien gemäß dem Stand der Technik.

Gegenstand der Erfindung sind organische Elektrolumineszenzvorrichtungen, enthaltend Kathode, Anode und mindestens eine emittierende Schicht, dadurch gekennzeichnet, dass die emittierende Schicht
a) 1 bis 20 Gew.% mindestens einer Verbindung gemäß Formel (1) enthält, 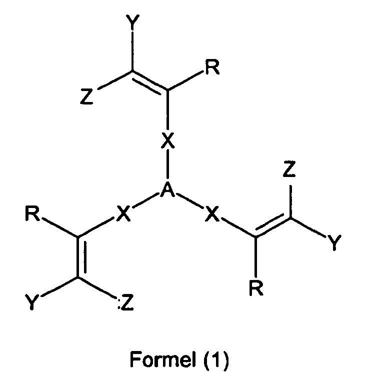 wobei für die Symbole und Indizes gilt:
   - A: ist bei jedem Auftreten Stickstoff, Phosphor, P=O, P=S, Arsen, As=O, As=S, Antimon, Sb=O oder Sb=S;
   - X: ist bei jedem Auftreten gleich oder verschieden ein bivalentes aromatisches oder heteroaromatisches Ringsystem mit 2 bis 60 C-Atomen, das mit einem oder mehreren Resten R¹ substituiert sein kann;
   - Y: ist bei jedem Auftreten gleich oder verschieden ein monovalentes aromatisches oder heteroaromatisches Ringsystem mit 2 bis 60 C-Atomen, das mit einem oder mehreren Resten R¹ substituiert sein kann, mit der Maßgabe, dass Y keine substituierten oder unsubstituierten Aminogruppen enthalten darf;
   - Z: ist bei jedem Auftreten gleich oder verschieden Y oder CN oder eine geradkettige, verzweigte oder cyclische Alkylgruppe mit 1 bis 40 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können;
   - R: ist bei jedem Auftreten gleich oder verschieden H, CN oder eine geradkettige, verzweigte oder cyclische Alkylgruppe mit 1 bis 40 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können;
   - R¹: ist bei jedem Auftreten gleich oder verschieden H, F, Cl, Br, I, CN, NO₂, eine geradkettige, verzweigte oder cyclische Alkyl-, Alkoxy- oder Thioalkoxygruppe mit 1 bis 40 C-Atomen, die mit einem oder mehreren Resten R² substituiert sein kann, wobei eine oder mehrere nicht benachbarte CH₂-Gruppen durch -R²C=CR²-, -C=C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können, oder ein aromatisches oder heteroaromatisches Ringsystem, das durch einen oder mehrere Reste R² substituiert sein kann, oder eine Aryloxy- oder Heteroaryloxygruppe mit 1 bis 40 aromatischen C-Atomen, die durch einen oder mehrere Reste R² substituiert sein kann, oder eine Kombination aus zwei bis fünf dieser Systeme; dabei können zwei oder mehrere Substituenten R¹ auch miteinander ein mono- oder polycyclisches, aliphatisches oder aromatisches Ringsystem bilden; mit der Maßgabe, dass R¹ keine Cyclohexylidenmethin-Gruppe darstellt;
   - R²: ist bei jedem Auftreten gleich oder verschieden H oder ein aliphatischer oder aromatischer Kohlenwasserstoffrest mit 1 bis 20 C-Atomen; und
b) 80 bis 99 Gew.% mindestens eines Hostmaterials enthält, welches ausgewählt ist aus den Klassen der Oligoarylene, enthaltend Naphthalin, Anthracen und/oder Pyren, der Oligoarylenvinylene, der Ketone, der Phosphinoxide oder der Sulfoxide.

Ein aromatisches Ringsystem im Sinne dieser Erfindung enthält 6 bis 60 C-Atome. Ein heteroaromatisches Ringsystem im Sinne dieser Erfindung enthält mindestens 2 C-Atome und mindestens 1 Heteroatom, bevorzugt ausgewählt aus Stickstoff, Schwefel und/oder Sauerstoff, mit der Maßgabe, dass die Summe der C-Atome und Heteroatome mindestens 5 ergibt. Unter einem aromatischen oder heteroaromatischen Ringsystem im Sinne dieser Erfindung soll ein System verstanden werden, das nicht notwendigerweise nur aromatische bzw. heteroaromatische Gruppen enthält, sondern in dem auch mehrere aromatische oder heteroaromatische Gruppen durch eine kurze, nicht-aromatische Einheit (weniger als 10 % der von H verschiedenen Atome, bevorzugt weniger als 5 % der von H verschiedenen Atome), wie z. B. ein sp³-hybridisiertes C-, N- oder O-Atom, unterbrochen sein können. So sollen also beispielsweise auch Systeme wie 9,9'-Spirobifluoren, 9,9-Diarylfluoren, Triarylamin, Diarylether, etc. als aromatische Ringsysteme im Sinne dieser Anmeldung verstanden werden.

Bevorzugt ist die Gruppe X so gewählt, dass die Konjugation zwischen dem zentralen Atom A und der Doppelbindung nicht unterbrochen ist.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 2 bis 60 C-Atomen, welches noch jeweils mit den oben genannten Resten R¹ substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Diphenylether, Triphenylamin, Dihydrophenanthren, Dihydropyren, Tetrahydropyren, cis- oder trans-Indenofluoren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt sind organische Elektrolumineszenzvorrichtungen, bei denen das Symbol A bei jedem Auftreten für Stickstoff, Phosphor oder P=O steht, besonders bevorzugt für Stickstoff.

Bevorzugt sind weiterhin organische Elektrolumineszenzvorrichtungen, bei denen das Symbol X bei jedem Auftreten gleich oder verschieden für eine bivalente Aryl- oder Heteroarylgruppe mit 3 bis 40 C-Atomen, die mit einem bis vier Resten R¹ substituiert sein kann, steht; besonders bevorzugt steht X bei jedem Auftreten gleich oder verschieden für eine bivalente Arylgruppe mit 6 bis 20 C-Atomen, die mit einem oder zwei Resten R¹ substituiert sein kann.

Bevorzugt sind weiterhin organische Elektrolumineszenzvorrichtungen, bei denen das Symbol Y gleich oder verschieden bei jedem Auftreten für eine monovalente Aryl- oder Heteroarylgruppe mit 3 bis 40 C-Atomen steht, die mit einem bis vier Resten R¹ substituiert sein kann; besonders bevorzugt steht Y bei jedem Auftreten gleich oder verschieden für eine monovalente Arylgruppe mit 6 bis 20 C=Atomen, die mit einem oder zwei Resten R¹ substituiert sein kann.

Bevorzugt sind weiterhin organische Elektrolumineszenzvorrichtungen, bei denen das Symbol Z, gleich oder verschieden bei jedem Auftreten für Y steht.

Bevorzugt sind weiterhin organische Elektrolumineszenzvorrichtungen, bei denen das Symbol R gleich oder verschieden bei jedem Auftreten für H oder eine Methylgruppe steht, besonders bevorzugt für H.

Weiterhin bevorzugt sind Verbindungen gemäß Formel (1), in denen Y und Z, die an eine Alkengruppe binden, für dasselbe monovalente aromatische Ringsystem stehen und bevorzugt auch gleich substituiert sind.
Besonders bevorzugt sind Verbindungen gemäß Formel (1), in denen alle Einheiten Y und Z gleich gewählt sind.
Nochmals weiterhin bevorzugt sind Verbindungen gemäß Formel (1), in denen alle Einheiten X gleich gewählt sind.
Besonders bevorzugt sind Verbindungen gemäß Formel (1), die symmetrisch aufgebaut sind und die eine dreizählige Drehachse (C3) aufweisen, insbesondere solche, in denen alle Einheiten X und alle Einheiten Y und Z und alle Substituenten R, R¹ und R² jeweils gleich gewählt sind.

In einer weiteren bevorzugten Ausführungsform der Erfindung stehen die Symbole Y und Z jeweils gleich oder verschieden bei jedem Auftreten für eine Aryl- oder Heteroarylgruppe mit 2 bis 40 C-Atomen, die mit R¹ substituiert sein kann, und bilden entweder über einen Substituenten R¹ oder über eine direkte Bindung ein Ringsystem miteinander. Besonders bevorzugt stehen Y und Z für die gleiche Aryl- oder Heteroarylgruppe, ganz besonders bevorzugt für Benzol. Weiterhin bevorzugt sind Y und Z über eine direkte Bindung oder eine Brücke R¹ mit 1 bis 3 Brückenatomen verbunden.

Beispiele für bevorzugte Verbindungen gemäß Formel (1) sind die im Folgenden abgebildeten Beispiele 1 bis 33, die mit R¹ substituiert oder unsubstituiert sein können.

| | | |
|---|---|---|
| 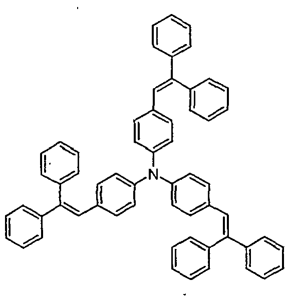 | 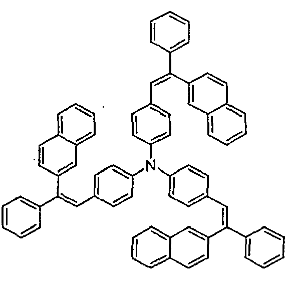 | 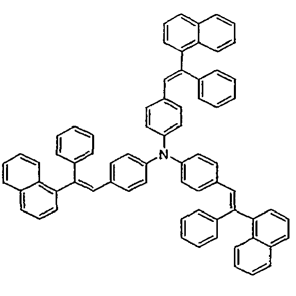 |
| Beispiel 1 | Beispiel 2 | Beispiel 3 |
| 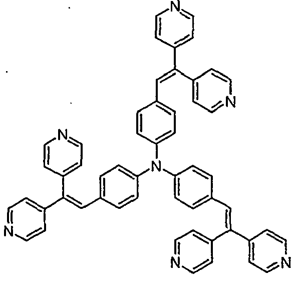 | 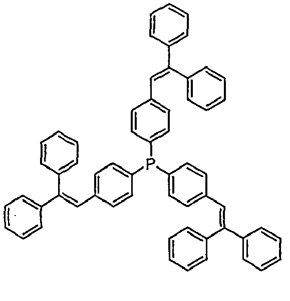 | 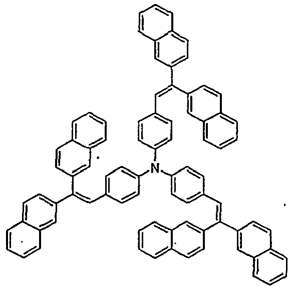 |
| Beispiel 4 | Beispiel 5 | Beispiel 6 |
| 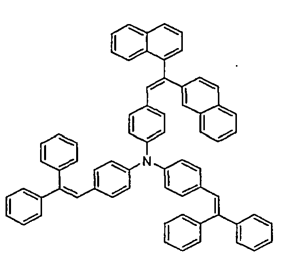 | 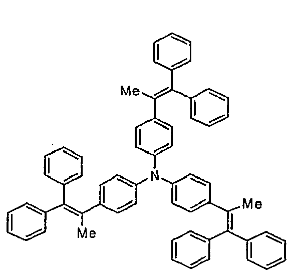 | 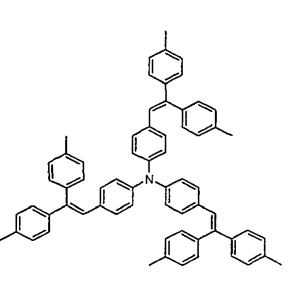 |
| Beispiel 7 | Beispiel 8 | Beispiel 9 |
| 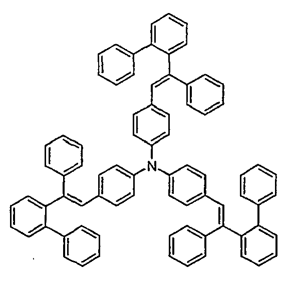 | 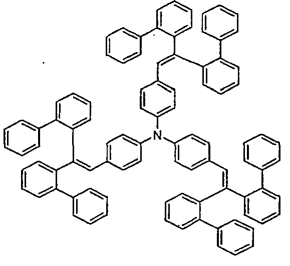 | 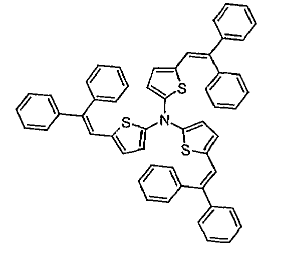 |
| Beispiel 10 | Beispiel 11 | Beispiel 12 |
| 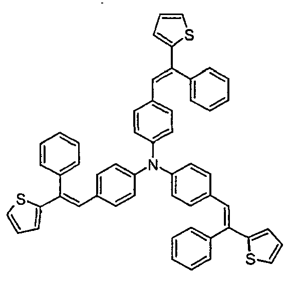 | 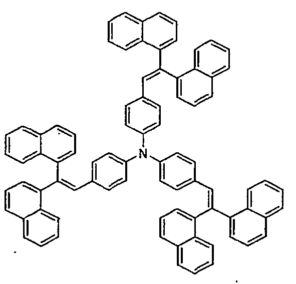 | 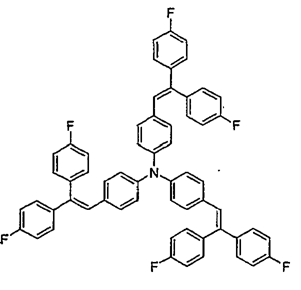 |
| Beispiel 13 | Beispiel 14 | Beispiel 15 |
| 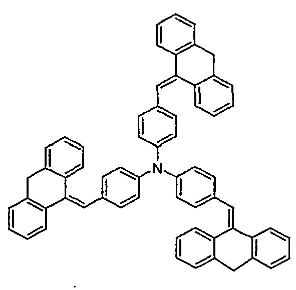 | 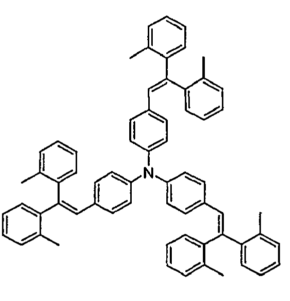 | 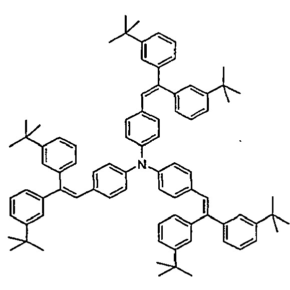 |
| Beispiel 16 | Beispiel 17 | Beispiel 18 |
| 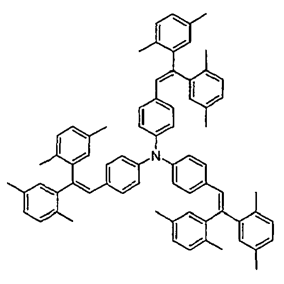 | 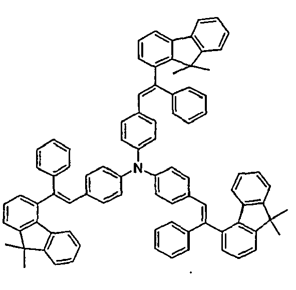 | 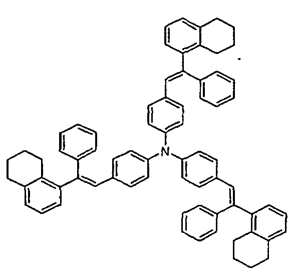 |
| Beispiel 19 | Beispiel 20 | Beispiel 21 |
| 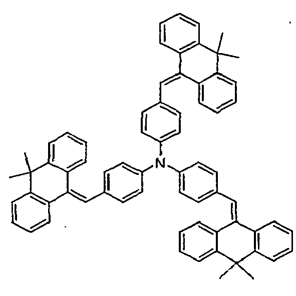 | 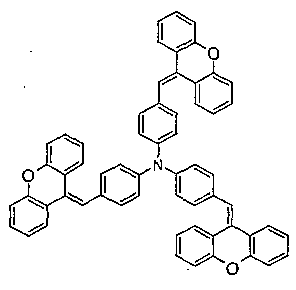 | 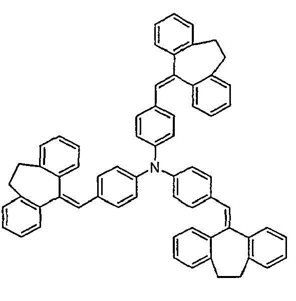 |
| Beispiel 22 | Beispiel 23 | Beispiel 24 |
| 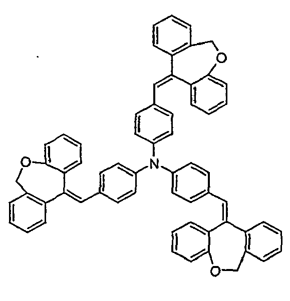 | 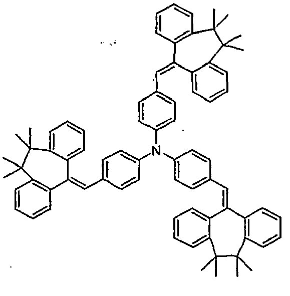 | 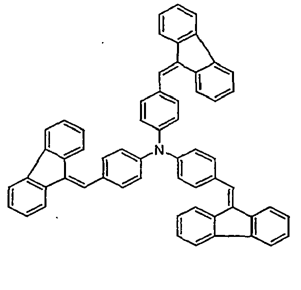 |
| Beispiel 25 | Beispiel 26 | Beispiel 27 |
| 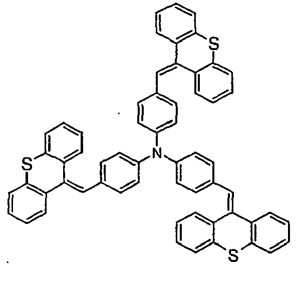 | 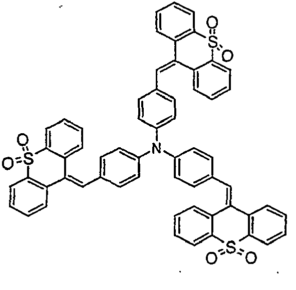 | 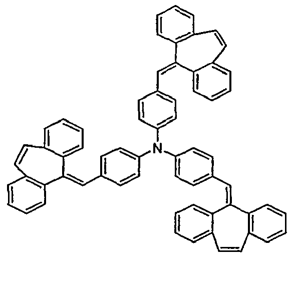 |
| Beispiel 28 | Beispiel 29 | Beispiel 30 |
| 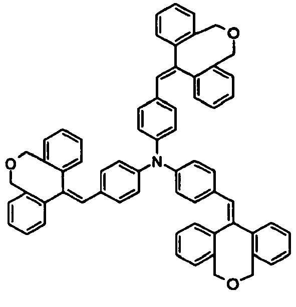 | 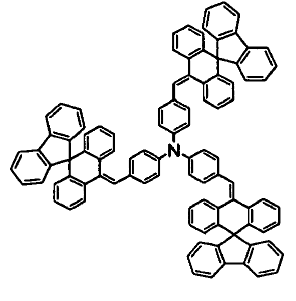 | 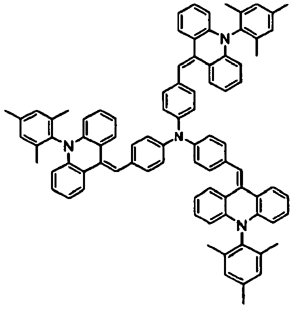 |
| Beispiel 31 | Beispiel 32 | Beispiel 33 |

Verbindungen gemäß Formel (1), in denen die Symbole Y und Z jeweils gleich oder verschieden bei jedem Auftreten für eine Aryl- oder Heteroarylgruppe stehen und über einen Substituenten R¹ oder über eine direkte Bindung ein Ringsystem miteinander bilden, sind neu und daher ebenfalls ein Gegenstand der Erfindung.

Die Verbindung gemäß Formel (1) wird in der emittierenden Schicht als Mischung mit mindestens einem Hostmaterial eingesetzt. Dabei ist es bevorzugt, wenn die Verbindung gemäß Formel (1) in der Mischung die emittierende Verbindung (der Dotand) ist und wenn das Hostmaterial nicht oder nur in geringerem Anteil emittiert.

Bevorzugte Hostmaterialien sind organische Verbindungen, deren Emission kürzerwellig ist als die der Verbindung gemäß Formel (1) bzw oder die überhaupt nicht im sichtbaren Bereich emittieren.

Als Hostmaterialien kommen verschiedene Stoffklassen in Frage. Bevorzugte Hostmaterialien sind ausgewählt aus den Klassen der Oligo-arylene (z. B. 2,2',7,7'-tetraphenyl-spirobifluoren gemäß EP 676461 oder Dinaphthylanthracen), der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911) oder der elektronenleitenden Verbindungen, insbesondere Ketone, Phosphinoxide, Sulfoxide, etc. (z. B. gemäß der nicht offen gelegten Patentanmeldung DE 102004008304.5). Besonders bevorzugte Hostmaterialien sind ausgewählt aus den Klassen der Oligoarylene, enthaltend Naphthalin, Anthracen und/oder Pyren, der Oligo-arylenvinylene, der Ketone, der Phosphinoxide und der Sulfoxide.

Der Anteil der Verbindung gemäß Formel (1) in der Mischung beträgt zwischen 1 und 20 Gew.%, bevorzugt zwischen 1 und 10 Gew.%.
Entsprechend beträgt der Anteil des Hostmaterials in der Mischung zwischen 80 und 99 Gew.%, bevorzugt zwischen 90 und 99 Gew.%.

Weiterhin bevorzugt sind organische Elektrolumineszenzvorrichtungen, dadurch gekennzeichnet, dass mehrere emittierende Verbindungen in derselben Schicht oder in unterschiedlichen Schichten verwendet werden, wovon mindestens eine dieser Verbindungen eine Struktur gemäß Formel (1) aufweist. Besonders bevorzugt weisen diese Verbindungen insgesamt mehrere Emissionsmaxima zwischen 380 nm und 750 nm auf, so dass insgesamt weiße Emission resultiert, d. h. außer der Verbindung gemäß Formel (1) wird noch mindestens eine weitere emittierende Verbindung verwendet, die fluoreszieren oder phosphoreszieren kann und die gelbes, orange oder rotes Licht emittiert. Ein solcher Aufbau ist beispielsweise in WO 05/011013 beschrieben.

Außer der emittierenden Schicht kann die organische Elektrolumineszenzvorrichtung noch weitere Schichten enthalten. Diese können beispielsweise sein:
Lochinjektionsschicht, Lochtransportschicht, Elektronentransportschicht und/oder Elektroneninjektionsschicht. Es sei aber an dieser Stelle darauf hingewiesen, dass nicht notwendigerweise jede dieser Schichten vorhanden sein muß.
So werden insbesondere bei Verwendung von Verbindungen gemäß Formel (1) mit elektronenleitenden Hostmaterialien weiterhin sehr gute Ergebnisse erhalten, wenn die organische Elektrolumineszenzvorrichtung keine separate Elektronentransportschicht enthält und die emittierende Schicht direkt an die Elektroneninjektionsschicht oder an die Kathode grenzt. Ebenfalls kann es bevorzugt sein, wenn die organische Elektrolumineszenzvorrichtung keine separate Lochtransportschicht enthält und die emittierende Schicht direkt an die Lochinjektionsschicht oder an die Anode grenzt. Weiterhin kann es bevorzugt sein, wenn die Verbindung gemäß Formel (1) nicht nur als Dotand in der emittierenden Schicht, sondern auch zusätzlich als lochleitende Verbindung (als Reinsubstanz oder als Mischung) in der Lochtransportschicht verwendet wird.
Bevorzugte Materialien für die Elektronentransportschicht sind Metallkomplexe, enthaltend Aluminium oder Gallium, polypodale Metallkomplexe (z. B. gemäß WO 04/081017), Ketone, Phosphinoxide oder Sulfoxide (z. B. gemäß der nicht offen gelegten Patentanmeldung DE 102004008304.5). Besonders bevorzugte Materialien für die Elektronentransportschicht sind Ketone oder Phosphinoxide.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z.B. durch Spincoating, oder mit einem beliebigen Druckverfahren, wie z.B. Siebdruck, Flexodruck oder Offsetdruck, besonders bevorzugt aber LITI (Light Induced Thermal Imaging, Thermotransferdruck) oder Ink-Jet Druck (Tintenstrahl-Druck), hergestellt werden.

Die oben beschriebenen emittierenden Vorrichtungen weisen folgende überraschende Vorteile gegenüber dem Stand der Technik auf:
1. Die Effizienz entsprechender Vorrichtungen wird höher im Vergleich zu Systemen gemäß dem Stand der Technik.
2. Die Stabilität entsprechender Vorrichtungen wird höher im Vergleich zu Systemen gemäß dem Stand der Technik, was sich vor allem in einer deutlich höheren Lebensdauer zeigt.
3. Die Betriebsspannung wird verringert. Dadurch erhöht sich die Leistungseffizienz. Dies gilt insbesondere, wenn ein Phosphinoxid als Hostmaterial verwendet wird.
4. Die Verbindungen lassen sich gut und ohne erhebliche Zersetzung sublimieren und aufdampfen, sind dadurch leichter zu verarbeiten und deshalb besser für die Verwendung in OLEDs geeignet als Materialien gemäß dem Stand der Technik, insbesondere als Materialien, die Stilbeneinheiten enthalten, die an der Doppelbindung nicht weiter substituiert sind.

Die Erfindung wird durch die nachfolgenden Beispiele näher erläutert, ohne sie dadurch einschränken zu wollen.

### Beispiele:

### Beispiel 1 : Synthese des Dotanden D1

Die nachfolgende Synthese wurde gemäß WO 02/10093 und, sofern nicht anders angegeben, unter einer Schutzgasatmosphäre durchgeführt. Die Edukte wurden von ALDRICH (Lösemittel, Palladium(II)acetat, Dimethylglycin, Eisen(III)chlorid, Tris-(4-bromphenyl)amin, 1,1-Diphenylethen) bezogen.
Ein Gemisch aus 24.1 g (50 mmol) Tris-(4-bromphenyl)amin, 39.7 ml (225 mmol) 1,1-Diphenylethen, 337 mg (1.5 mmol) Palladium(II)acetat, 1.55 g (15 mmol) N,N-Dimethylglycin, 243 mg (1.5 mmol) Eisen(III)chlorid und 37.8 g (450 mmol) Natriumhydrogencarbonat in 500 ml NMP wurde langsam unter gutem Rühren auf 140 °C erhitzt und anschließend 16 h bei dieser Temperatur gerührt. Nach Erkalten wurden 500 ml Dichlormethan und 1000 ml Wasser zugesetzt. Die organische Phase wurde abgetrennt und fünfmal mit 500 ml Wasser gewaschen. Nach Trocknen über Natriumsulfat wurde die organische Phase zur Trockene eingeengt, der so erhaltene gelbe Feststoff wurde viermal aus DMSO umkristallisiert, dann zweimal mit 500 ml Ethanol unter Rückfluss ausgerührt und anschließend dreimal im Vakuum (T = 315 °C, p = 5 x 10⁻⁵ mbar) sublimiert. Es wurden 33.5 g (entsprechend 85.9 % d. Th.) des Produkts mit einer Reinheit größer 99.9 % n. HPLC erhalten.
¹H-NMR (CDCl₃): δ [ppm] = 7.35 - 7.21 (m, 30H, C₆H₅), 6.88 (s, 3H, vinyl-CH), 6.83, 6.75 (d, ³J_{HH} = 8.7 Hz, 12H, C₆H₄).

### Beispiel 2: Herstellung der OLEDs

Die Herstellung von OLEDs erfolgte nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wurde.

In den folgenden Beispielen 3 bis 7 werden die Ergebnisse verschiedener OLEDs vorgestellt. Der grundlegende Aufbau, die verwendeten Materialien und Schichtdicken, außer der emittierenden Schicht und der Elektronentransportschicht, waren in den Beispielen 3 bis 6 zur besseren Vergleichbarkeit identisch. Analog dem o. g. allgemeinen Verfahren wurden OLEDs mit folgendem Aufbau erzeugt (wobei die unten aufgeführte Lochtransportschicht nicht für Beispiel 7 zutrifft):

| | |
|---|---|
| Lochinjektionsschicht (HIL) | 60 nm PEDOT (aus Wasser aufgeschleudert; bezogen von H. C. Starck, Goslar, Deutschland; Poly(3,4-ethylendioxy-2,5-thiophen)) |
| Lochtransportschicht (HTM) | 20 nm NaphDATA (aufgedampft; bezogen von SynTec, Wolfen, Deutschland; 4,4',4"-Tris(N-1-naphthyl-N-phenyl-amino)-triphenylamin) |
| Lochtransportschicht (HTM) | 20 nm S-TAD (aufgedampft; hergestellt nach WO 99/12888; 2,2',7,7'-Tetrakis(diphenylamino)-spiro-9,9'-bifluoren) |
| Emissionschicht (EML) | siehe Tabelle 1 für Materialien, Konzentration und Schichtdicken |
| Elektronenleiter (ETL) | siehe Tabelle 1 für Materialien und Schichtdicken |
| Ba-Al (Kathode) | 3 nm Ba, darauf 150 nm Al |

In Beispiel 7 wurden als Lochtransportmaterialien außer den oben genannten NPB (N-Naphthyl-N-phenyl-4,4'-diaminobiphenyl) und die Verbindung **HTM1** (2,2',7,7'-Tetrakis(di-para-tolylamino)spiro-9,9'-bifluoren) verwendet.

Diese noch nicht optimierten OLEDs wurden standardmäßig charakterisiert; hierfür wurden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit der OLED bei einer konstanten Stromdichte von 10 mA/cm² auf die Hälfte gesunken ist.

In Tabelle 1 sind die Ergebnisse einiger OLEDs (Beispiele 3 bis 6) zusammengefasst, wobei jeweils die Zusammensetzung der EML und der ETL inklusive der Schichtdicken mit aufgeführt ist. Die EMLs enthalten als emittierende Materialien gemäß Formel (1) den Dotanden **D1** (gemäß Beispiel 1). Als Vergleichsbeispiele dienen OLEDs, die als emittierende Verbindungen den Dotanden **D2** gemäß dem oben genannten Stand der Technik bzw. nur das Hostmaterial enthalten. Als Hostmaterialien dienen die im Folgenden abgebildeten Verbindungen **H1** bis **H4.** Als Elektronentransportmaterialien dienen **ETM1** (AlQ₃, bezogen von SynTec, Tris(chinolinato)aluminium(III)), **ETM2** (Bis(9,9'-spirobifluoren-2-yl)keton gemäß WO 04/093207 oder **ETM3** (Bis(9,9`-spirobifluoren-2-yl)phenylphosphinoxid gemäß WO 05/003253).
In Tabelle 2 sind die Ergebnisse weiterer OLEDs (Beispiel 7) zusammengefasst, die durch Variation der Lochtransportschichten auf bessere Effizienz und Lebensdauer optimiert wurden.
Zur besseren Übersichtlichkeit sind die entsprechenden Strukturformeln der verwendeten Dotanden und Hostmaterialien im Folgenden dargestellt:

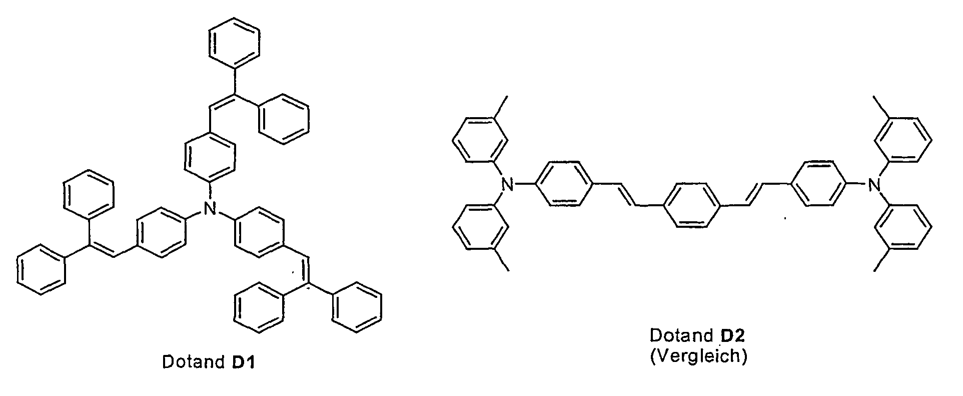

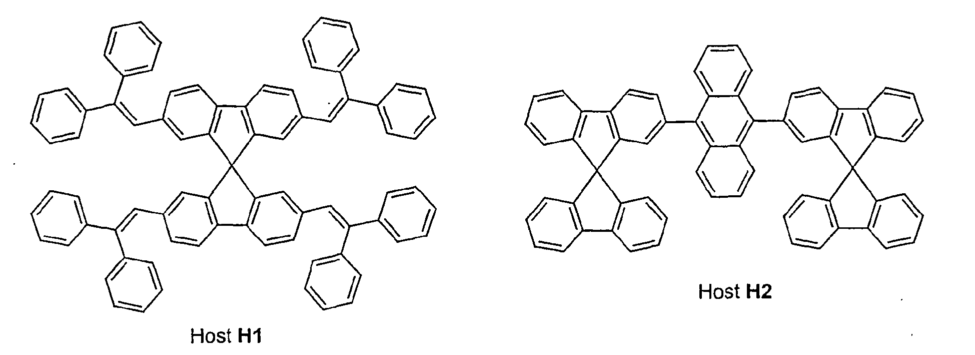

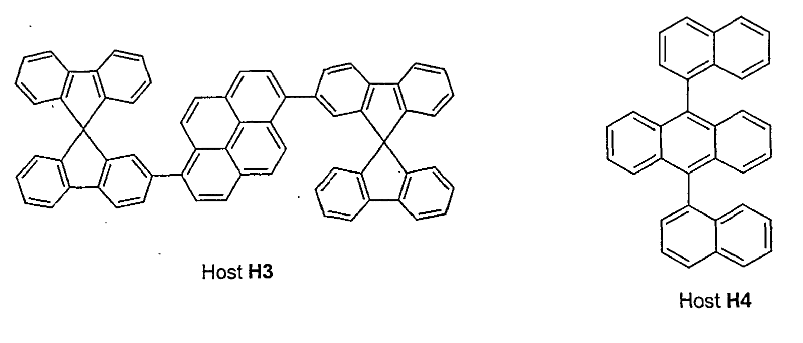

**Tabelle 1**

| Beispiel | EML | ETL | Max Effizienz (cd/A) | Spannung (V) bei 100cd/m² | CIE | Lebensdauer (h) |
|---|---|---|---|---|---|---|
| **Beispiel 3a** | **H1** | **EMT1** | 4.2 | 5.8 | x=0.17; y=0.26 | 1200 |
| (Vergleich) | (30 nm) | (20 nm) | | | | |
| **Beispiel 3b** | **H1 : D2** (5 %) | **ETM1** | 4.9 | 6.3 | x=0.17; y=0.31 | 1000 |
| (Vergleich) | (30 nm) | (20 nm) | | | | |
| **Beispiel 3c** | **H1: D1** (2 %) | **EMT1** | 4.5 | 5.4 | x=0.17; y=0.30 | 2200 |
| | (30 nm) | (20 nm) | | | | |
| **Beispiel 3d** | **H1 : D1** (5 %) | **EMT1** | 4.8 | 5.2 | x=0.18; y=0.33 | 4000 |
| | (30 nm) | (20 nm) | | | | |
| **Beispiel 3e** | **H1 : D1** (5 %) | **EMT2** | 4.5 | 4.8 | x=0.17; y=0.29 | 4500 |
| | (30 nm) | (20 nm) | | | | |
| **Beispiel 3f** | **H1 : D1** (5 %) | **EMT2** | 5.0 | 5.0 | x=0.17; y=0.30 | 3800 |
| | (30 nm) | (30 nm) | | | | |
| **Beispiel 3g** | **H1 : D1** (5 %) | **EMT2** | 5.2 | 5.2 | x=0.17; y=0.31 | 3600 |
| | (30 nm) | (40 nm) | | | | |
| **Beispiel 4a** | **H2** | **EMT1** | 3.3 | 6.5 | x=0.15; y=0.15 | 700 |
| (Vergleich) | (30 nm) | (20 nm) | | | | |
| **Beispiel 4b** | **H2 : D1** (2 %) | **EMT1** | 4.5 | 5.7 | x=0.17; y=0.25 | 1800 |
| | (30 nm) | (20 nm) | | | | |
| **Beispiel 4c** | **H2 : D1** (5 %) | **EMT1** | 4.8 | 5.3 | x=0.18; y=0.30 | 3800 |
| | (30 nm) | (20 nm) | | | | |
| **Beispiel 5a** | **H3** | **EMT1** | 3.7 | 5.8 | x=0.17; y=0.26 | 1400 |
| (Vergleich) | (30 nm) | (20 nm) | | | | |
| **Beispiel 5b** | **H3 : D2** (5 %) | **ETM1** | 5.4 | 6.5 | x=0.19; y=0.37 | 1000 |
| (Vergleich) | (30 nm) | (20 nm) | | | | |
| **Beispiel 5c** | **H3 : D1** (2 %) | **EMT1** | 4.7 | 5.7 | x=0.16; y=0.28 | 2900 |
| | (30 nm) | (20 nm) | | | | |
| **Beispiel 5d** | **H3 : D1** (5 %) | **EMT1** | 5.5 | 5.5 | x=0.17; y=0.31 | 5400 |
| | (30 nm) | (20 nm) | | | | |
| **Beispiel 6a** | **H4** | **EMT1** | 1.1 | 5.8 | x=0.17; y=0.19 | 3100 |
| (Vergleich) | (30 nm) | (20 nm) | | | | |
| **Beispiel 6b** | **H4 : D1** (2 %) | **EMT1** | 6.5 | 5.7 | x=0.16; y=0.28 | 7000 |
| | (30 nm) | (20 nm) | | | | |
| **Beispiel 6c** | **H4 : D1** (5 %) | **EMT1** | 7.9 | 5.3 | x=0.17; y=0.31 | 15000 |
| | (30 nm) | (20 nm) | | | | |
| **Beispiel 6d** | **H4 : D1** (5 %) | **ETM2** | 6.5 | 5.0 | x=0.17; y=0.28 | 16500 |
| | (30 nm) | (20 nm) | | | | |
| **Beispiel 6e** | **H4 : D1** (5 %) | **ETM2** | 6.7 | 5.2 | x=0. 17; y=0.31 | 15000 |
| | (30 nm) | (30 nm) | | | | |
| **Beispiel 6f** | **H4 : D1** (5 %) | **ETM2** | 7.4 | 5.4 | x=0.17; y=0.32 | 14000 |
| | (30 nm) | (40 nm) | | | | |
| **Beispiel 6g** | **H4 : D1** (5%) | **ETM3** | 4.7 | 5.1 | x=0.17; y=0.30 | 20500 |
| | (30 nm) | (20 nm) | | | | |
| **Beispiel 6h** | **H4 : D1** (5%) | **ETM3** | 4.9 | 5.4 | x=0.17; y=0.31 | 20000 |
| | (30 nm) | (30 nm) | | | | |

**Tabelle 2**

| Beispiel | HTL1 | HTL2 | EML | ETL | Max Effizienz (cd/A) | Spannung (V) bei 100cd/m² | CIE | Lebensdauer (h) |
|---|---|---|---|---|---|---|---|---|
| **Beispiel 7a** | **HTM1** | **S-TAD** | **H4** : D1 (5%) | **EML1** | 7.6 | 4.4 | x=0.17; y=0.28 | 17000 |
| | (20 nm) | (20 nm) | (30 nm) | (20 nm) | | | | |
| **Beispiel 7b** | **NaphDATA** | **NPB** | **H4 :** D1 (5%) | **ETM1** | 10.5 | 5.3 | x=0.17; y=0.28 | 15500 |
| | (20 nm) | (20 nm) | (30 nm) | (20 nm) | | | | |
| **Beispiel 7c** | **HTM1** | **NPB** | **H4** : D1 (5%) | **EMT1** | 8.1 | 4.8 | x=0.17; y=0.28 | 20000 |
| | (20 nm) | (20 nm) | (30 nm) | (20 nm) | | | | |

### Beispiel 8: Vergleich der thermischen Stabilität

Wie in Beispiel 1 beschrieben, wurde der Dotand **D1** (Molgewicht = 780 g/mol) dreimal im Vakuum bei 315 °C sublimiert. Die Sublimation verlief rückstandsfrei und ohne Anzeichen von Zersetzung, überprüft mit ¹H-NMR und HPLC. Lagerversuche bei 330 °C für 160 h ergaben ebenfalls keinen Hinweis auf eine thermisch induzierte Zersetzung des Dotanden **D1.**
Im Vergleich dazu besitzt der Dotand **D2** (Vergleichsbeispiel gemäß dem Stand der Technik, Molgewicht = 673 g/mol) eine deutlich geringere Stabilität. Die Sublimation von **D2** mit einer Anfangsreinheit nach ¹H-NMR und HPLC von größer 99.9 % bei T = 330 °C und p = 5 x 10⁻⁵ mbar ergab nach 2 h:
- ca. 69 Gew.% der eingesetzten Menge in Form eines verharzten, in gängigen organischen Lösungsmittels nur schwer löslichen Rückstands,
- ca. 30 Gew.% eines gelben Sublimats,
- Spuren an farblosem Bis(3-methylphenyl)amin, bestimmt nach ¹H-NMR.
Das gelbe Sublimat war nicht einheitlich. Es bestand nach ¹H-NMR und HPLC-Analkytik aus einem Gemisch an *trans,trans-, cis,trans-* und *cis,cis-*Konfigurationsisomeren von **D2.** Lagerversuche bei 330 °C für 160 h führten zu einer nahezu vollständigen Zersetzung (Verharzung) des Dotanden **D2.**

Diese Ergebnisse zeigen, dass der Dotand **D1** eine ausgezeichnete Langzeitstabilität aufweist und daher für den industriellen Einsatz bestens geeignet ist. Insbesondere ist die Langzeittemperaturstabilität des Dotanden **D1** deutlich höher als die des Vergleichsdotanden **D2** gemäß dem Stand der Technik.

Zusammenfassend kann gesagt werden, dass OLEDs, enthaltend emittierende Verbindungen gemäß Formel (1), eine deutlich höhere Effizienz bei vergleichbaren oder niedrigeren Spannungen und bei deutlich längerer Lebensdauer aufweisen als Materialien gemäß dem Stand der Technik, wie man leicht Tabelle 1 entnehmen kann. Außerdem lassen sie sich durch die höhere thermische Stabilität deutlich leichter verarbeiten als Materialien gemäß dem Stand der Technik, wie in Beispiel 8 beschrieben. Daher eignen sich diese Verbindungen besser für die Verwendung in OLEDs als Materialien gemäß dem Stand der Technik.

## Patentansprüche

1. Organische Elektrolumineszenzvorrichtungen, enthaltend Kathode, Anode und mindestens eine emittierende Schicht, **dadurch gekennzeichnet, dass** die emittierende Schicht
a) 1 bis 20 Gew.% mindestens einer Verbindung gemäß Formel (1) enthält, 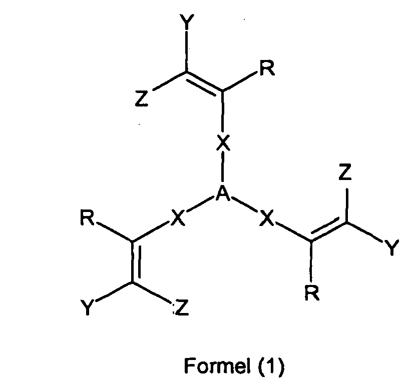 wobei für die Symbole und Indizes gilt:
A ist bei jedem Auftreten Stickstoff, Phosphor, P=O, P=S, Arsen, As=O, As=S, Antimon, Sb=O oder Sb=S;
X ist bei jedem Auftreten gleich oder verschieden ein bivalentes aromatisches oder heteroaromatisches Ringsystem mit 2 bis 60 C-Atomen, das mit einem oder mehreren Resten R¹ substituiert sein kann;
Y ist bei jedem Auftreten gleich oder verschieden ein monovalentes aromatisches oder heteroaromatisches Ringsystem mit 2 bis 60 C-Atomen, das mit einem oder mehreren Resten R¹ substituiert sein kann, mit der Maßgabe, dass Y keine substituierte oder unsubstituierte Aminogruppe enthalten darf;
Z ist bei jedem Auftreten gleich oder verschieden Y oder CN oder eine geradkettige, verzweigte oder cyclische Alkylgruppe mit 1 bis 40 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können;
R ist bei jedem Auftreten gleich oder verschieden H, CN oder eine geradkettige, verzweigte oder cyclische Alkylgruppe mit 1 bis 40 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können;
R¹ ist bei jedem Auftreten gleich oder verschieden H, F, Cl, Br, I, CN, NO₂, eine geradkettige, verzweigte oder cyclische Alkyl-, Alkoxy- oder Thioalkoxygruppe mit 1 bis 40 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können, oder ein aromatisches oder heteroaromatisches Ringsystem, das mit einem oder mehreren Resten R² substituiert sein kann, oder eine Aryloxy- oder Heteroaryloxygruppe mit 1 bis 40 aromatischen C-Atomen, die durch einen oder mehrere Reste R² substituiert sein kann, oder eine Kombination aus zwei bis fünf dieser Systeme; dabei können zwei oder mehrere Substituenten R¹ auch miteinander ein mono- oder polycyclisches, aliphatisches oder aromatisches Ringsystem bilden; mit der Maßgabe, dass R¹ keine Cyclohexylidenmethin-Gruppe darstellt;
R² ist bei jedem Auftreten gleich oder verschieden H oder ein aliphatischer oder aromatischer Kohlenwasserstoffrest mit 1 bis 20 C-Atomen; und
b) 80 bis 99 Gew.% mindestens eines Hostmaterials enthält, welches ausgewählt ist aus den Klassen der Oligo-arylene, enthaltend Naphthalin, Anthracen und/oder Pyren, der Oligo-arylenvinylene, der Ketone, der Phosphinoxide oder der Sulfoxide.

2. Organische Elektrolumineszenzvorrichtungen gemäß Anspruch 1, **dadurch gekennzeichnet, dass** das Symbol A für Stickstoff, Phosphor oder P=O steht.

3. Organische Elektrolumineszenzvorrichtungen gemäß Anspruch 1 und/oder 2, **dadurch gekennzeichnet, dass** das Symbol X gleich oder verschieden bei jedem Auftreten für eine bivalente Aryl- oder Heteroarylgruppe mit 3 bis 40 C-Atomen steht, die mit einem bis vier Resten R¹ substituiert sein kann.

4. Organische Elektrolumineszenzvorrichtungen gemäß einem oder mehreren der Ansprüche 1 bis 3, **dadurch gekennzeichnet, dass** das Symbol Y gleich oder verschieden bei jedem Auftreten für eine bivalente Aryl- oder Heteroarylgruppe mit 3 bis 40 C-Atomen steht, die mit einem bis vier Resten R¹ substituiert sein kann.

5. Organische Elektrolumineszenzvorrichtungen gemäß einem oder mehreren der Ansprüche 1 bis 4, **dadurch gekennzeichnet, dass** das Symbol Z gleich oder verschieden bei jedem Auftreten Y ist.

6. Organische Elektrolumineszenzvorrichtungen gemäß einem oder mehreren der Ansprüche 1 bis 5, **dadurch gekennzeichnet, dass** das Symbol R gleich oder verschieden bei jedem Auftreten H oder eine Methylgruppe ist.

7. Organische Elektrolumineszenzvorrichtungen gemäß einem oder mehreren der Ansprüche 1 bis 6, **dadurch gekennzeichnet, dass** in Verbindungen gemäß Formel (1) jeweils Y und Z, die an eine Alkengruppe binden, für dasselbe monovalente aromatische Ringsystem stehen.

8. Organische Elektrolumineszenzvorrichtungen gemäß Anspruch 7, **dadurch gekennzeichnet, dass** in Verbindungen gemäß Formel (1) alle Einheiten Y und Z gleich gewählt sind.

9. Organische Elektrolumineszenzvorrichtungen gemäß einem oder mehreren der Ansprüche 1 bis 8, **dadurch gekennzeichnet, dass** in Verbindungen gemäß Formel (1) alle Einheiten X gleich gewählt sind.

10. Organische Elektrolumineszenzvorrichtungen gemäß Anspruch 9, **dadurch gekennzeichnet, dass** die Verbindungen gemäß Formel (1) symmetrisch aufgebaut sind und C₃-Symmetrie aufweisen.

11. Organische Elektrolumineszenzvorrichtungen gemäß einem oder mehreren der Ansprüche 1 bis 10, **dadurch gekennzeichnet, dass** die Verbindungen gemäß Formel (1) ausgewählt sind aus den folgenden Beispielstrukturen 1 bis 27:
| | | |
|---|---|---|
| 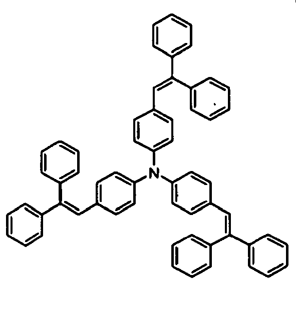 | 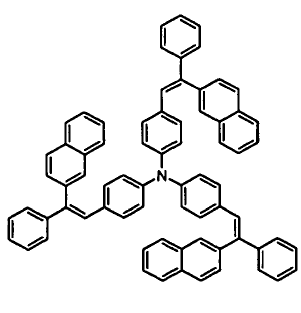 | 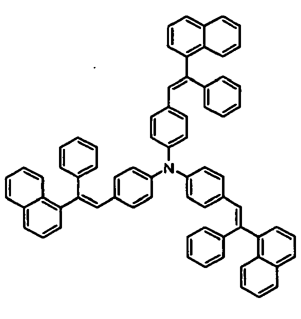 |
| Beispiel 1 | Beispiel 2 | Beispiel 3 |
| 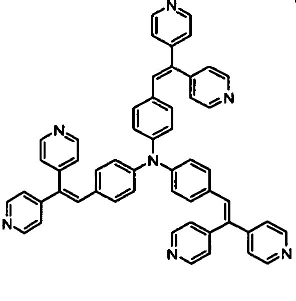 | 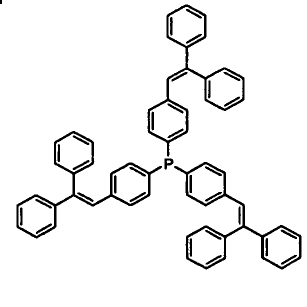 | 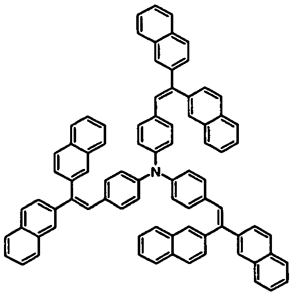 |
| Beispiel 4 | Beispiel 5 | Beispiel 6 |
| 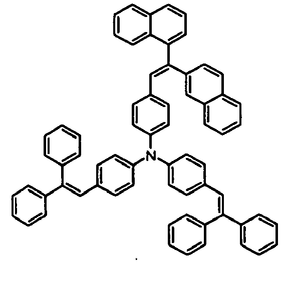 | 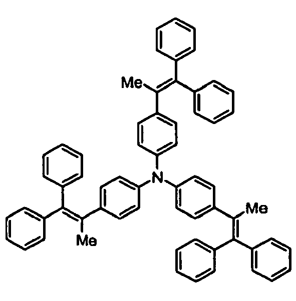 | 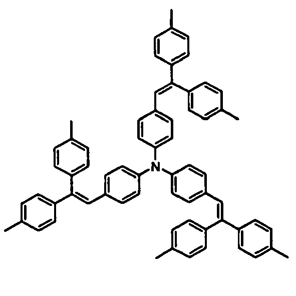 |
| Beispiel 7 | Beispiel 8 | Beispiel 9 |
| 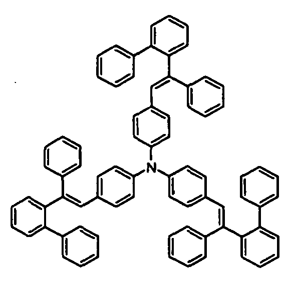 | 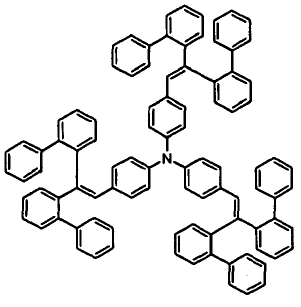 | 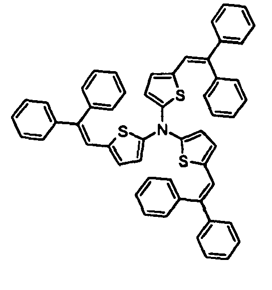 |
| Beispiel 10 | Beispiel 11 | Beispiel 12 |
| 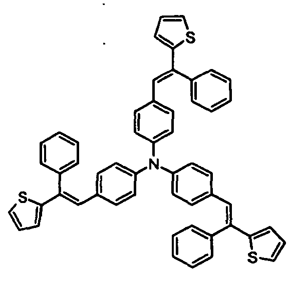 | 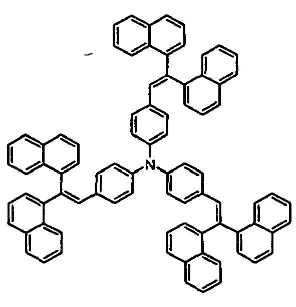 | 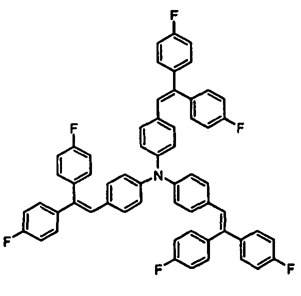 |
| Beispiel 13 | Beispiel 14 | Beispiel 15 |
| 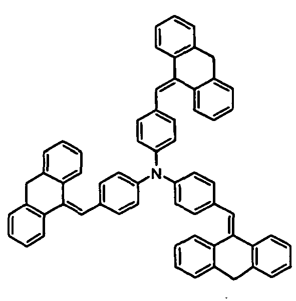 | 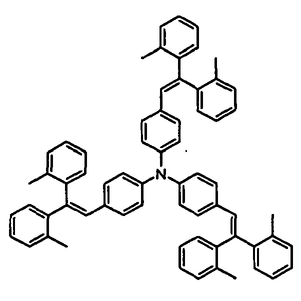 | 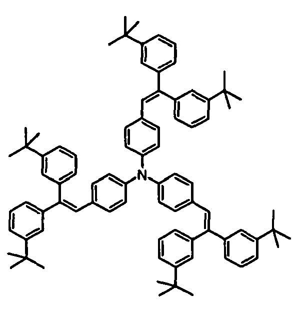 |
| Beispiel 16 | Beispiel 17 | Beispiel 18 |
| 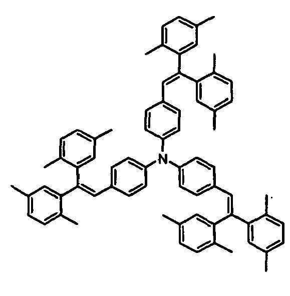 | 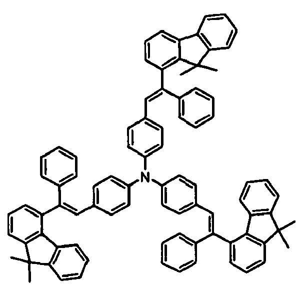 | 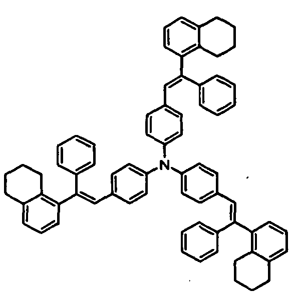 |
| Beispiel 19 | Beispiel 20 | Beispiel 21 |
| 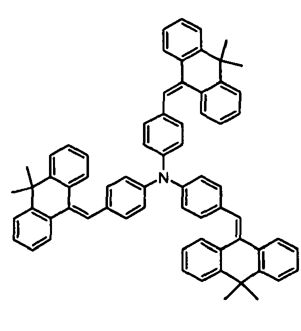 | 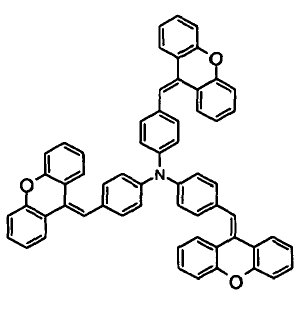 | 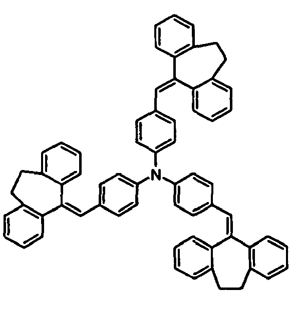 |
| Beispiel 22 | Beispiel 23 | Beispiel 24 |
| 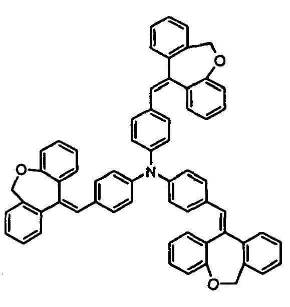 | 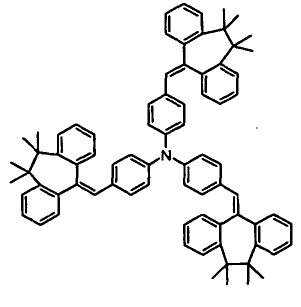 | 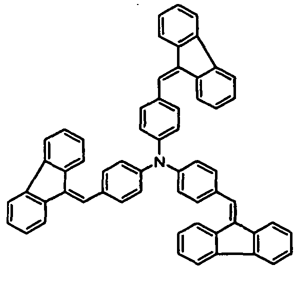 |
| Beispiel 25 | Beispiel 26 | Beispiel 27 |

12. Organische Elektrolumineszenzvorrichtungen gemäß einem oder mehreren der Ansprüche 1 bis 11, **dadurch gekennzeichnet, dass** die Hostmaterialien organische Verbindungen sind, deren Emission kürzerwellig ist als die der Verbindung gemäß Formel (1) oder die überhaupt nicht im sichtbaren Bereich emittieren.

13. Organische Elektrolumineszenzvorrichtungen gemäß einem oder mehreren der Ansprüche 1 bis 12, **dadurch gekennzeichnet, dass** mehrere emittierende Verbindungen in derselben Schicht oder in unterschiedlichen Schichten verwendet werden, wovon mindestens eine dieser Verbindungen eine Struktur gemäß Formel (1) aufweist und dass diese Verbindungen insgesamt mehrere Emissionsmaxima zwischen 380 nm und 750 nm aufweisen, so dass insgesamt weiße Emission resultiert.

14. Organische Elektrolumineszenzvorrichtungen gemäß einem oder mehreren der Ansprüche 1 bis 13, **dadurch gekennzeichnet, dass** außer der emittierenden Schicht noch eine oder mehrere Schichten, ausgewählt aus Lochinjektionsschichten, Lochtransportschichten, Elektronentransportschichten und/oder Elektroneninjektionsschichten, anwesend sind.

15. Organische Elektrolumineszenzvorrichtungen gemäß Anspruch 14, **dadurch gekennzeichnet, dass** mindestens eine Elektronentransportschicht anwesend ist, enthaltend mindestens eine Keto-Verbindung, ein Phosphinoxid oder ein Sulfoxid.

## Claims

1. Organic electroluminescent devices comprising cathode, anode and at least one emitting layer, **characterised in that** the emitting layer comprises
a) 1 to 20% by weight of at least one compound of the formula (1) 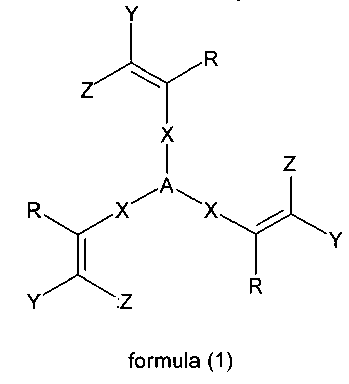 where the following applies to the symbols and indices:
A is on each occurrence nitrogen, phosphorus, P=O, P=S, arsenic, As=O, As=S, antimony, Sb=O or Sb=S;
X is on each occurrence, identically or differently, a divalent aromatic or heteroaromatic ring system having 2 to 60 C atoms, which may be substituted by one or more radicals R¹;
Y is on each occurrence, identically or differently, a monovalent aromatic or heteroaromatic ring system having 2 to 60 C atoms, which may be substituted by one or more radicals R¹, with the proviso that Y cannot contain a substituted or unsubstituted amino group;
Z is on each occurrence, identically or differently, Y or CN or a straight-chain, branched or cyclic alkyl group having 1 to 40 C atoms, which may be substituted by one or more radicals R², where one or more non-adjacent CH₂ groups may be replaced by -R²C=CR²-, -C≡C-, Si(R²)₂, Ge(R²)₂, Sn(R²)₂, C=O, C=S, C=Se, C=NR², -O-, -S- or -CONR²- and where one or more H atoms may be replaced by F, Cl, Br, I, CN or NO₂;
R is on each occurrence, identically or differently, H, CN or a straight-chain, branched or cyclic alkyl group having 1 to 40 C atoms, which may be substituted by one or more radicals R², where one or more non-adjacent CH₂ groups may be replaced by -R²C=CR²-, -C≡C-, Si(R²)₂, Ge(R²)₂, Sn(R²)₂, C=O, C=S, C=Se, C=NR², -O-, -S- or -CONR²- and where one or more H atoms may be replaced by F, Cl, Br, I, CN or NO₂;
R¹ is on each occurrence, identically or differently, H, F, Cl, Br, I, CN, NO₂, a straight-chain, branched or cyclic alkyl, alkoxy or thioalkoxy group having 1 to 40 C atoms, which may be substituted by one or more radicals R², where one or more non-adjacent CH₂ groups may be replaced by -R²C=CR²-, -C≡C-, Si(R²)₂, Ge(R²)₂, Sn(R²)₂, C=O, C=S, C=Se, C=NR², -O-, -S- or -CONR²- and where one or more H atoms may be replaced by F, Cl, Br, I, CN or NO₂, or an aromatic or heteroaromatic ring system, which may be substituted by one or more radicals R², or an aryloxy or heteroaryloxy group having 1 to 40 aromatic C atoms, which may be substituted by one or more radicals R², or a combination of two to five of these systems; two or more substituents R¹ here may also form a mono- or polycyclic, aliphatic or aromatic ring system with one another; with the proviso that R¹ does not represent a cyclohexylidenemethine group;
R² is on each occurrence, identically or differently, H or an aliphatic or aromatic hydrocarbon radical having 1 to 20 C atoms; and
b) 80 to 99% by weight of at least one host material which is selected from the classes of the oligoarylenes comprising naphthalene, anthracene and/or pyrene, the oligoarylenevinylenes, the ketones, the phosphine oxides and the sulfoxides.

2. Organic electroluminescent devices according to Claim 1, **characterised in that** the symbol A stands for nitrogen, phosphorus or P=O.

3. Organic electroluminescent devices according to Claim 1 and/or 2, **characterised in that** the symbol X, identically or differently on each occurrence, stands for a divalent aryl or heteroaryl group having 3 to 40 C atoms, which may be substituted by one to four radicals R¹.

4. Organic electroluminescent devices according to one or more of Claims 1 to 3, **characterised in that** the symbol Y, identically or differently on each occurrence, stands for a divalent aryl or heteroaryl group having 3 to 40 C atoms, which may be substituted by one to four radicals R¹.

5. Organic electroluminescent devices according to one or more of Claims 1 to 4, **characterised in that** the symbol Z, identically or differently on each occurrence, is Y.

6. Organic electroluminescent devices according to one or more of Claims 1 to 5, **characterised in that** the symbol R, identically or differently on each occurrence, is H or a methyl group.

7. Organic electroluminescent devices according to one or more of Claims 1 to 6, **characterised in that**, in compounds of the formula (1), Y and Z, which are bonded to an alkene group, each stand for the same monovalent aromatic ring system.

8. Organic electroluminescent devices according to Claim 7, **characterised in that**, in compounds of the formula (1), all units Y and Z are selected identically.

9. Organic electroluminescent devices according to one or more of Claims 1 to 8, **characterised in that**, in compounds of the formula (1), all units X are selected identically.

10. Organic electroluminescent devices according to Claim 9, **characterised in that** the compounds of the formula (1) have a symmetrical structure and C₃ symmetry.

11. Organic electroluminescent devices according to one or more of Claims 1 to 10, **characterised in that** the compounds of the formula (1) are selected from the following example structures 1 to 27:
| | | |
|---|---|---|
| 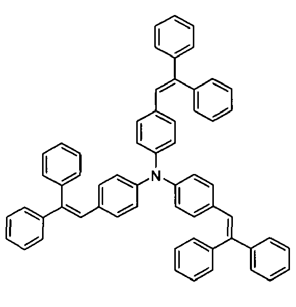 | 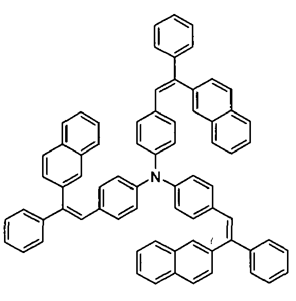 | 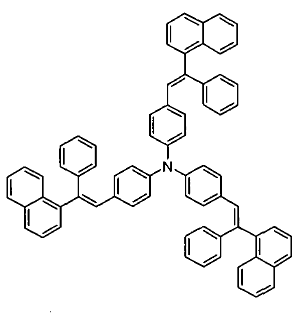 |
| Example 1 | Example 2 | Example 3 |
| 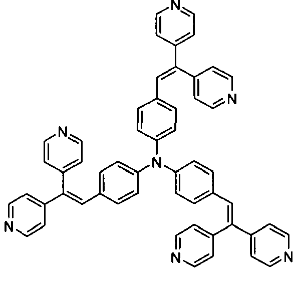 | 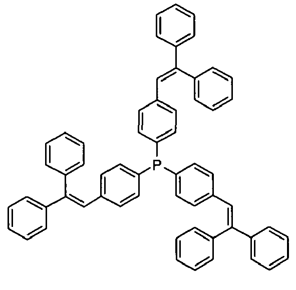 | 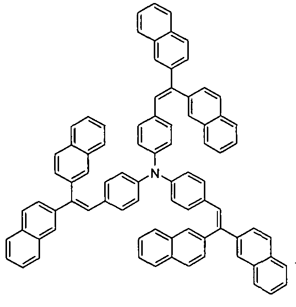 |
| Example 4 | Example 5 | Example 6 |
| 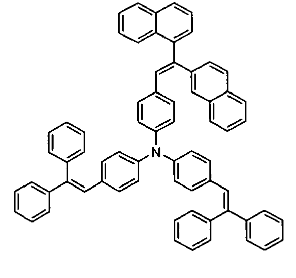 | 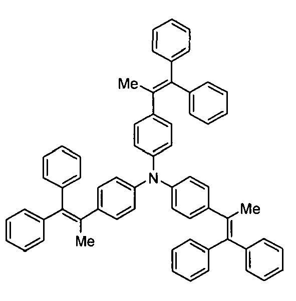 | 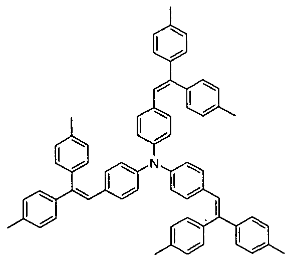 |
| Example 7 | Example 8 | Example 9 |
| 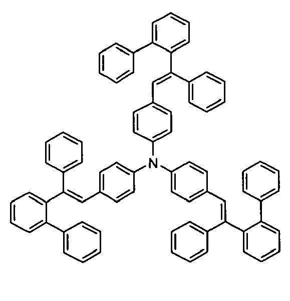 | 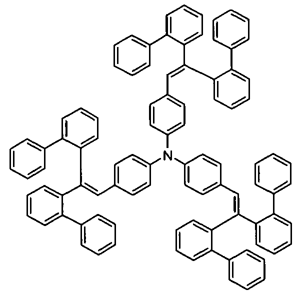 | 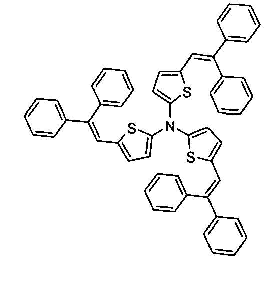 |
| Example 10 | Example 11 | Example 12 |
| 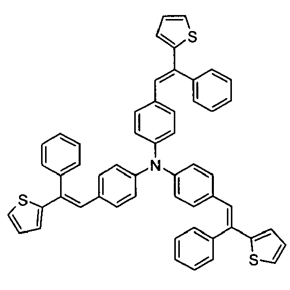 | 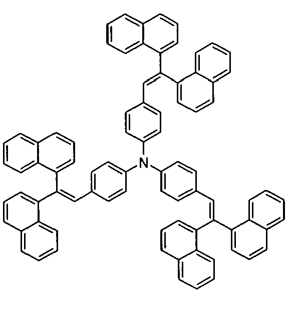 | 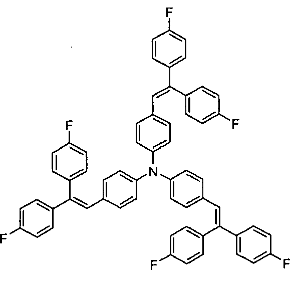 |
| Example 13 | Example 14 | Example 15 |
| 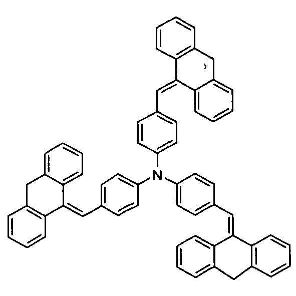 | 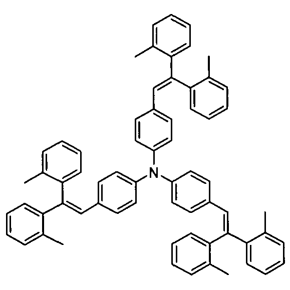 | 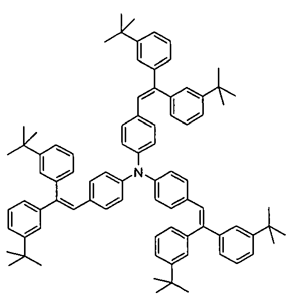 |
| Example 16 | Example 17 | Example 18 |
| 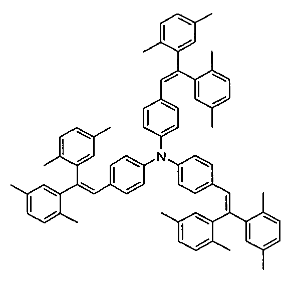 | 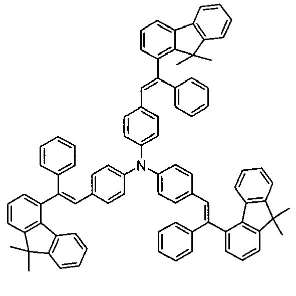 | 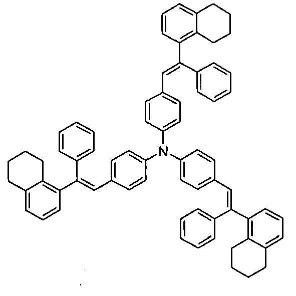 |
| Example 19 | Example 20 | Example 21 |
| 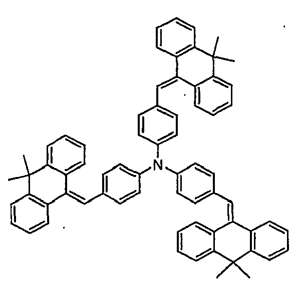 | 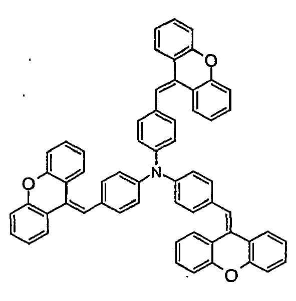 | 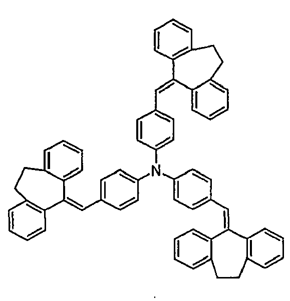 |
| Example 22 | Example 23 | Example 24 |
| 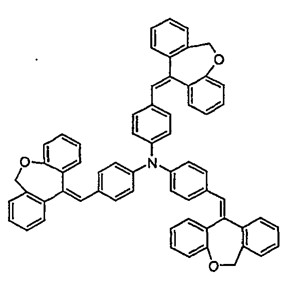 | 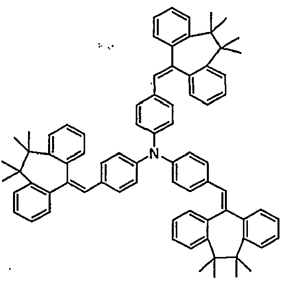 | 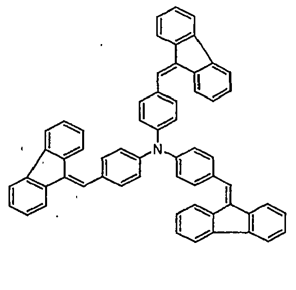 |
| Example 25 | Example 26 | Example 27 |

12. Organic electroluminescent devices according to one or more of Claims 1 to 11, **characterised in that** the host materials are organic compounds whose emission is of shorter wavelength than that of the compound of the formula (1) or which do not emit at all in the visible region.

13. Organic electroluminescent devices according to one or more of Claims 1 to 12, **characterised in that** a plurality of emitting compounds are used in the same layer or in different layers, where at least one of these compounds has a structure of the formula (1), and that these compounds have in total a plurality of emission maxima between 380 nm and 750 nm, resulting overall in white emission.

14. Organic electroluminescent devices according to one or more of Claims 1 to 13, **characterised in that**, apart from the emitting layer, one or more layers selected from hole-injection layers, hole-transport layers, electron-transport layers and/or electron-injection layers are also present.

15. Organic electroluminescent devices according to Claim 14, **characterised in that** at least one electron-transport layer comprising at least one keto compound, a phosphine oxide or a sulfoxide is present.

## Revendications

1. Dispositifs électroluminescents organiques comprenant une cathode, une anode et au moins une couche d'émission, **caractérisés en ce que** la couche d'émission comprend
a) 1 à 20% en poids d'au moins un composé de la formule (1) 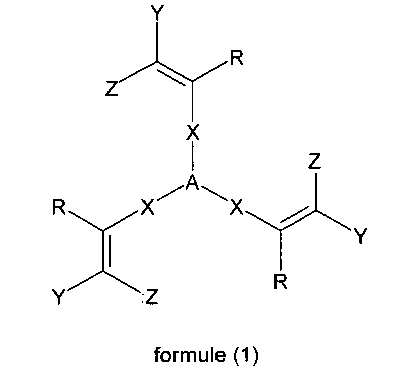 dans laquelle ce qui suit s'applique aux symboles et indices :
A est à chaque occurrence azote, phosphore, P=O, P=S, arsenic, As=O, As=S, antimoine, Sb=O ou Sb=S ;
X est à chaque occurrence, de façon identique ou différente, un système de cycle aromatique ou hétéroaromatique divalent ayant 2 à 60 atomes de C, qui peuvent être substitués par un ou plusieurs radicaux R¹;
Y est à chaque occurrence, de façon identique ou différente, un système de cycle aromatique ou hétéroaromatique monovalent ayant 2 à 60 atomes de C, qui peuvent être substitués par un ou plusieurs radicaux R¹, sous réserve que Y ne puisse pas contenir un groupe amino substitué ou non substitué ;
Z est à chaque occurrence, de façon identique ou différente, Y ou CN ou un groupe alkyle en chaîne droite, ramifié ou cyclique ayant 1 à 40 atomes de C, qui peuvent être substitués par un ou plusieurs radicaux R², dans lequel un ou plusieurs groupes CH₂ non adjacents peuvent être remplacés par -R²C=CR²-, -C≡C-, Si(R²)₂, Ge(R²)₂, Sn(R²)₂, C=O, C=S, C=Se, C=NR², -O-, -S- ou -CONR²- et dans lequel un ou plusieurs atomes de H peuvent être remplacés par F, CI, Br, I, CN ou NO₂ ;
R est à chaque occurrence, de façon identique ou différente, H, CN ou un groupe alkyle en chaîne droite, ramifié ou cyclique ayant 1 à 40 atomes de C, qui peuvent être substitués par un ou plusieurs radicaux R², dans lequel un ou plusieurs groupes CH₂ non adjacents peuvent être remplacés par -R²C=CR²-, -C≡C-, Si(R²)₂, Ge(R²)_{2,} Sn(R²)₂, C=O, C=S, C=Se, C=NR², -O-, -S- ou -CONR²- et dans lequel un ou plusieurs atomes de H peuvent être remplacés par F, CI, Br, I, CN ou NO₂ ;
R¹ est à chaque occurrence, de façon identique ou différente, H, F, CI, Br, I, CN, NO₂, un groupe alkyle, alcoxy ou thioalcoxy en chaîne droite, ramifié ou cyclique ayant 1 à 40 atomes de C, qui peuvent être substitués par un ou plusieurs radicaux R², dans lequel un ou plusieurs groupes CH₂ non adjacents peuvent être remplacés par -R²C=CR² -, -C≡C-, Si(R²)₂, Ge(R²)_{2,} Sn(R²)_{2,} C=O, C=S, C=Se, C=NR², -O-, -S- ou -CONR²- et dans lequel un ou plusieurs atomes de H peuvent être remplacés par F, CI, Br, I, CN ou NO₂, ou un système de cycle aromatique ou hétéroaromatique, qui peut être substitué par un ou plusieurs radicaux R², ou un groupe aryloxy ou hétéroaryloxy ayant 1 à 40 atomes de C aromatiques, qui peuvent être substitués par un ou plusieurs radicaux R², ou une combinaison de deux à cinq de ces systèmes ; deux ou plus de deux substituants R¹ peuvent ici également former un système de cycle aliphatique ou aromatique mono- ou polycyclique avec un autre ; sous réserve que R¹ ne représente pas un groupe cyclohexylidèneméthine ;
R² est à chaque occurrence, de façon identique ou différente, H ou un radical hydrocarbure aliphatique ou aromatique ayant 1 à 20 atomes de C ; et
b) 80 à 99% en poids d'au moins un matériau hôte qui est choisi parmi les classes des oligoarylènes comprenant naphtalène, anthracène et/ou pyrène, des oligoarylènevinylènes, des cétones, des oxydes de phosphine et des sulfoxydes.

2. Dispositifs électroluminescents organiques selon la revendication 1, **caractérisés en ce que** le symbole A représente azote, phosphore ou P=O.

3. Dispositifs électroluminescents organiques selon la revendication 1 et/ou 2, **caractérisés en ce que** le symbole X, de façon identique ou différente à chaque occurrence, représente un groupe aryle ou hétéroaryle divalent ayant 3 à 40 atomes de C, qui peuvent être substitués par un à quatre radicaux R¹.

4. Dispositifs électroluminescents organiques selon une ou plusieurs des revendications 1 à 3, **caractérisés en ce que** le symbole Y, de façon identique ou différente à chaque occurrence, représente un groupe aryle ou hétéroaryle divalent ayant 3 à 40 atomes de C, qui peuvent être substitués par un à quatre radicaux R¹.

5. Dispositifs électroluminescents organiques selon une ou plusieurs des revendications 1 à 4, **caractérisés en ce que** le symbole Z, de façon identique ou différente à chaque occurrence, est Y.

6. Dispositifs électroluminescents organiques selon une ou plusieurs des revendications 1 à 5, **caractérisés en ce que** le symbole R, de façon identique ou différente à chaque occurrence, est H ou un groupe méthyle.

7. Dispositifs électroluminescents organiques selon une ou plusieurs des revendications 1 à 6, **caractérisés en ce que**, dans les composés de la formule (1), Y et Z, qui sont liés à un groupe alcène, représentent chacun le même système de cycle aromatique monovalent.

8. Dispositifs électroluminescents organiques selon la revendication 7, **caractérisés en ce que**, dans les composés de la formule (1), toutes les unités Y et Z sont choisies de façon identique.

9. Dispositifs électroluminescents organiques selon une ou plusieurs des revendications 1 à 8, **caractérisés en ce que**, dans les composés de la formule (1), toutes les unités X sont choisies de façon identique.

10. Dispositifs électroluminescents organiques selon la revendication 9, **caractérisés en ce que** les composés de la formule (1) ont une structure symétrique et une symétrie C₃.

11. Dispositifs électroluminescents organiques selon une ou plusieurs des revendications 1 à 10, **caractérisés en ce que** les composés de la formule (1) sont choisis parmi les structures d'exemples suivantes 1 à 27:
| | | |
|---|---|---|
| 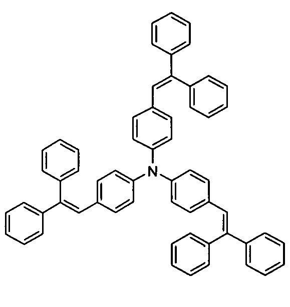 | 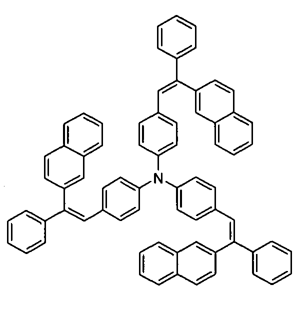 | 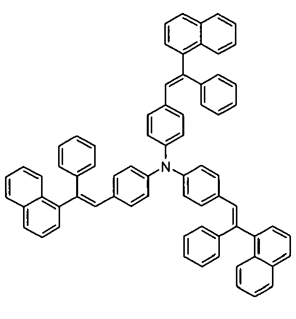 |
| Exemple 1 | Exemple 2 | Exemple 3 |
| 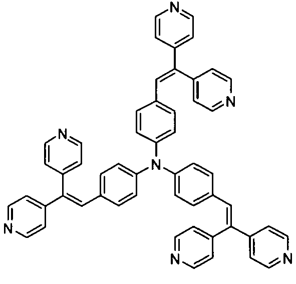 | 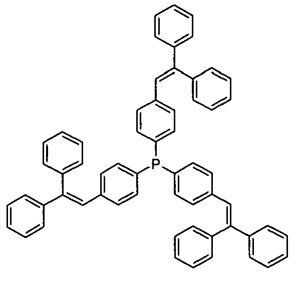 | 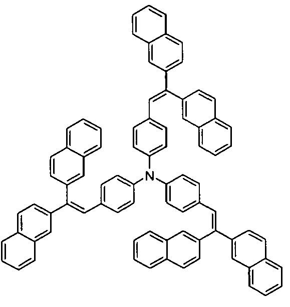 |
| Exemple 4 | Exemple 5 | Exemple 6 |
| 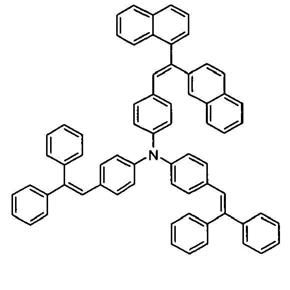 | 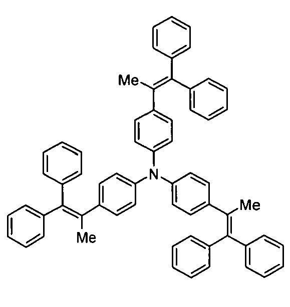 | 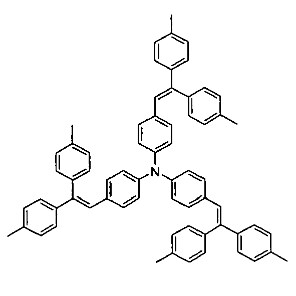 |
| Exemple 7 | Exemple 8 | Exemple 9 |
| 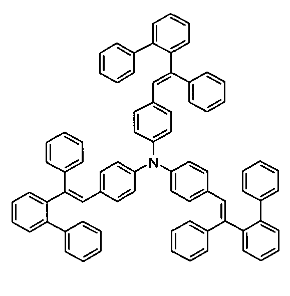 | 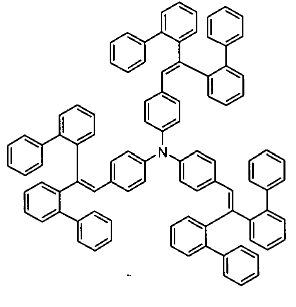 | 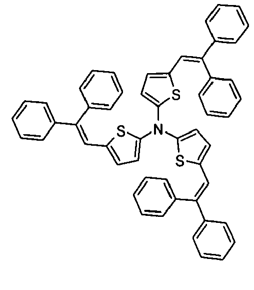 |
| Exemple 10 | Exemple 11 | Exemple 12 |
| 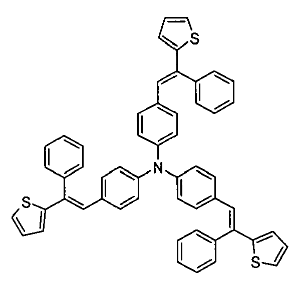 | 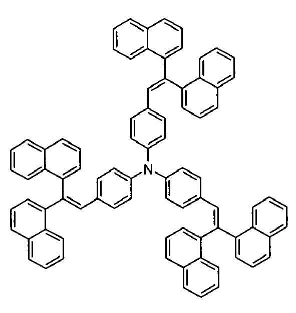 | 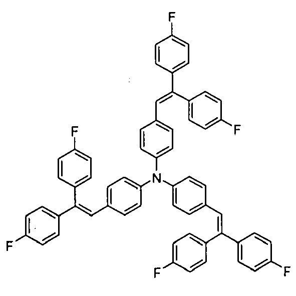 |
| Exemple 13 | Exemple 14 | Exemple 15 |
| 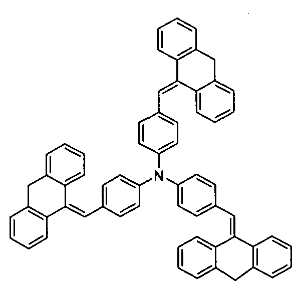 | 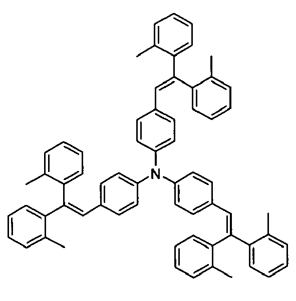 | 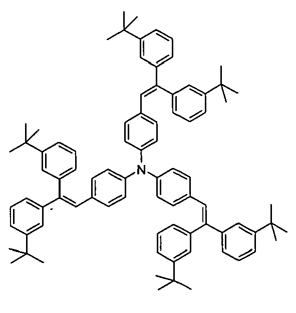 |
| Exemple 16 | Exemple 17 | Exemple 18 |
| 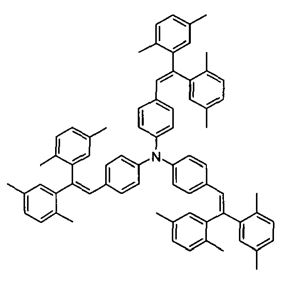 | 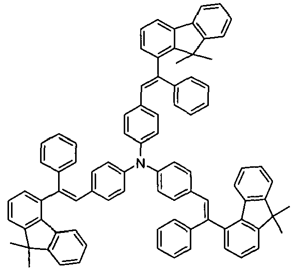 | 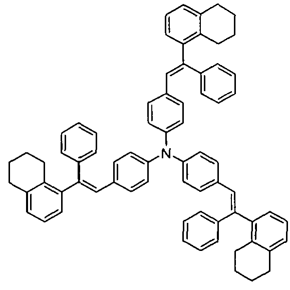 |
| Exemple 19 | Exemple 20 | Exemple 21 |
| 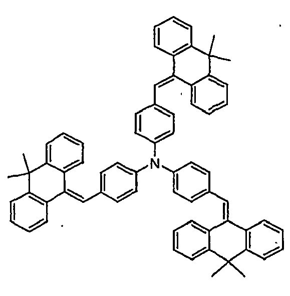 | 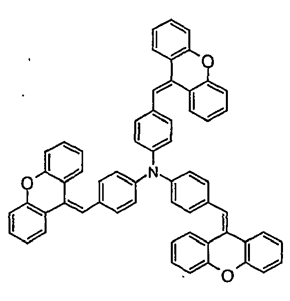 | 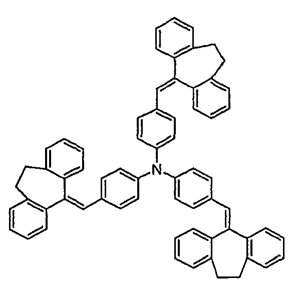 |
| Exemple 22 | Exemple 23 | Exemple 24 |
| 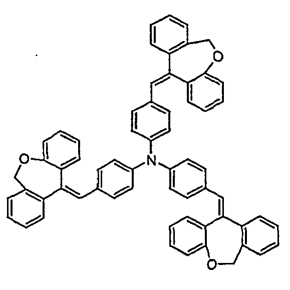 | 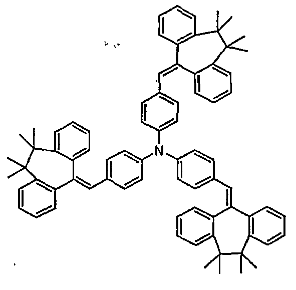 | 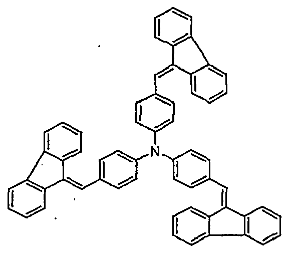 |
| Exemple 25 | Exemple 26 | Exemple 27 |

12. Dispositifs électroluminescents organiques selon une ou plusieurs des revendications 1 à 11, **caractérisés en ce que** les matériaux hôtes sont des composés organiques dont l'émission est de longueur d'onde plus courte que celle du composé de la formule (1) ou qui n'émettent pas du tout dans la région visible.

13. Dispositifs électroluminescents organiques selon une ou plusieurs des revendications 1 à 12, **caractérisés en ce qu'**une pluralité de composés d'émission sont utilisés dans la même couche ou dans des couches différentes, dans lesquels au moins un de ces composés a une structure de la formule (1), et **en ce que** ces composés ont au total une pluralité de maxima d'émission entre 380 nm et 750 nm, conduisant globalement à une émission blanche.

14. Dispositifs électroluminescents organiques selon une ou plusieurs des revendications 1 à 13, **caractérisés en ce que**, en plus de la couche d'émission, une ou plusieurs couches choisies parmi des couches d'injection de trous, des couches de transport de trous, des couches de transport d'électrons et/ou des couches d'injection d'électrons sont également présentes

15. Dispositifs électroluminescents organiques selon la revendication 14, **caractérisés en ce qu'**au moins une couche de transport d'électrons comprenant au moins un composé céto, un oxyde de phosphine ou un sulfoxyde est présente.
